## Europäisches Patentamt

## European Patent Office

## Office européen des brevets

(19)

(11) Veröffentlichungsnummer: **0 236 259**

**A2**

(12) # EUROPÄISCHE PATENTANMELDUNG

(21) Anmeldenummer: **87810004.9**

(22) Anmeldetag: **06.01.87**

(51) Int. Cl.⁴: **C 12 M 1/06**
**C 12 M 1/08**

(30) Priorität: **05.03.86 CH 904/86**

(43) Veröffentlichungstag der Anmeldung:
**09.09.87 Patentblatt 87/37**

(84) Benannte Vertragsstaaten: **DE FR GB IT**

(71) Anmelder: **Tschudin & Heid AG**
**Haupstrasse 35**
**CH-4437 Waldenburg (CH)**

(72) Erfinder: **Baerfuss, Achilles**
**Sonnenweg 5**
**CH-4104 Oberwil (CH)**

(74) Vertreter: **Eder, Carl E. et al**
**Patentanwaltsbüro EDER AG Münchensteinerstrasse 2**
**CH-4052 Basel (CH)**

(54) **Einrichtung zum Behandeln von Mikroorganismen.**

(57) Die Einrichtung besitzt eine Kammer (5), deren Innenraum (21) im einen Höhenbereich durch eine Trennwand (19) in zwei Teilräume (23, 25) unterteilt ist, und ein Förderorgan (63), mit dem flüssiges, Mikroorganismen enthaltendes Substrat (121) aus dem ersten Teilraum (23) durch den zweiten Teilraum (25) hindurch nach oben gefördert werden kann, so dass es über einen Überström-Rand (29) beim oberen Ende der Trennwand (19) wieder in den ersten Teilraum (23) zurückfällt. Für die Durchführung aerober, mikrobiologischer Prozesse wird mit einem Gasverteiler (51), zwischen dem Förderorgan (63) und dem Überström-Rand (29) sauerstoffhaltiges Gas in das umgewälzte Substrat (121) eingeleitet. Dadurch, dass das Gas erst in das Substrat eingeleitet wird, wenn dieses das Förderorgan (63) passiert hat, kann man auch sehr grosse Gasmengen pro Zeiteinheit zuführen, ohne die Förderwirkung des Förderorgans (63) zu behindern.

Fig.1

**Beschreibung**

Einrichtung zum Behandeln von Mikroorganismen

Die Erfindung betrifft eine Einrichtung gemäss dem Oberbegriff des Anspruchs 1.

Solche, aus der Schweizerpatentschriften 358 195 und 606 436 bekannte, als Fermenter dienende Einrichtungen weisen eine Kammer mit einem zylindrischen Mantel und einem sich nach unten konisch verjüngenden Boden auf. Im Innenraum der Kammer ist eine diesen in zwei Teilräume unterteilende Trennwand angeordnet. Diese ist an ihrem unteren Ende mit einem sich nach unten konisch verjüngenden Leitelement und einem Hals versehen, in dem das Schaufelrad einer Pumpe angeordnet ist. Im von der Trennwand umschlossenen, ersten Teilraum ist ein Luftverteiler angeordnet, der oberhalb des Schaufelrades in den genannten Teilraum mündet und mit einer Luftzuleitung verbunden ist.

Beim Betrieb einer solchen Einrichtung wälzt die Pumpe ein in der Kammer vorhandenes Substrat derart um, dass dieses vom ersten Teilraum in den zwischen der äusseren Wand der Kammer und der Trennwand vorhandenen, zweiten Teilraum hinein und durch diesen hindurch nach oben gepumpt wird, wo es über den vom oberen Rand der Trennwand gebildeten Überström-Rand wieder in den ersten Teilraum zurückfällt. Während dieser Umwälzung des Substrats wird diesem mit dem Luftverteiler die für einen aeroben, mikrobiologischen Prozess benötigte Luft zugeführt.

Bei grosser Luftzufuhr können sich im Substrat oberhalb des Schaufelrades und/oder bei diesem Luftsäcke bilden, die die Substrat-Umwälzung reduzieren oder sogar vorübergehend vollständig unterbrechen. Dabei kann es beispielsweise geschehen, dass die Substrat-Umwälzung in mehr oder weniger regelmässigen, in der Grösse von wenigen Minuten liegenden Zeitabständen unterbrochen wird. Bei einer derartigen, schwankenden und intermittierenden Umwälzung des Substrats wird nicht nur dessen Bewegung, sondern auch noch dessen Durchlüftung ungleichmässig, was sich ungünstig auf den Prozessablauf und die Produktqualität auswirkt.

Der Erfindung liegt nun die Aufgabe zugrunde, eine Einrichtung zu schaffen, die auch bei grossen Luftzufuhrmengen eine gleichmässige Umwälzung des Substrates und eine gleichmässige Gaseinleitung in dieses ermöglicht.

Diese Aufgabe wird durch eine Einrichtung der einleitend genannten Art gelöst, wobei die Einrichtung erfindungsgemäss durch die Merkmale des Anspruchs 1 gekennzeichnet ist. Vorteilhafte Ausgestaltungen der Einrichtung gehen aus den abhängigen Ansprüchen hervor.

Der Erfindungsgegenstand wird nun anhand eines in der Zeichnung dargestellten Ausführungsbeispiels erläutert. In der Zeichnung zeigen

die Figur 1 einen schematisierten Vertikalschnitt einer Einrichtung zum Kultivieren von Mikroorganismen und

die Figur 2 einen Ausschnitt aus der Figur 1 in grösserem Massstab.

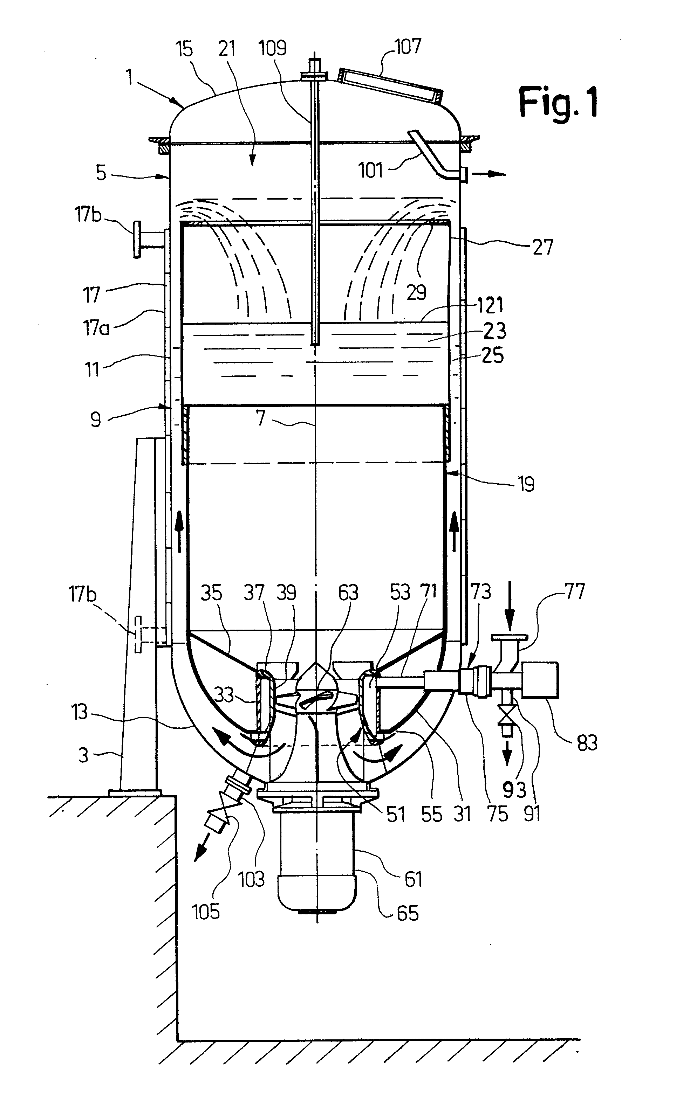

Die vereinfacht in der Figur 1 dargestellte, zum Behandeln, insbesondere Kultivieren und Entwickeln von Mikroorganismen in einem flüssigen Substrat dienende Einrichtung weist einen Reaktor 1 mit einem nur zum Teil angedeuteten Gestell 3 und einer von diesem gehaltenen Kammer 5 auf. Diese hat eine vertikale Achse 7 und eine zumindest im allgemeinen zu dieser rotationssymmetrische Wandung 9. Die letztere weist als Hauptbestandteil einen zylindrischen Abschnitt 11 auf, an den unten ein sich zum untern Kammerende hin an die Achse 7 annähernder, im Vertikalschnitt gebogener End- oder Bodenabschnitt 13 und oben ein lösbar befestigter Deckel 15 anschliesst. Der zylindrische Wandabschnitt 11 ist mit einer Vorrichtung 17 zum wahlweisen Kühlen oder Heizen versehen, die mindestens einen beispielsweise wendelförmigen Durchgang 17a für ein Kühl- bzw. Heizfluid sowie zwei Anschlüsse 17b zum Ein- bzw. Auslassen dieses Fluids besitzen.

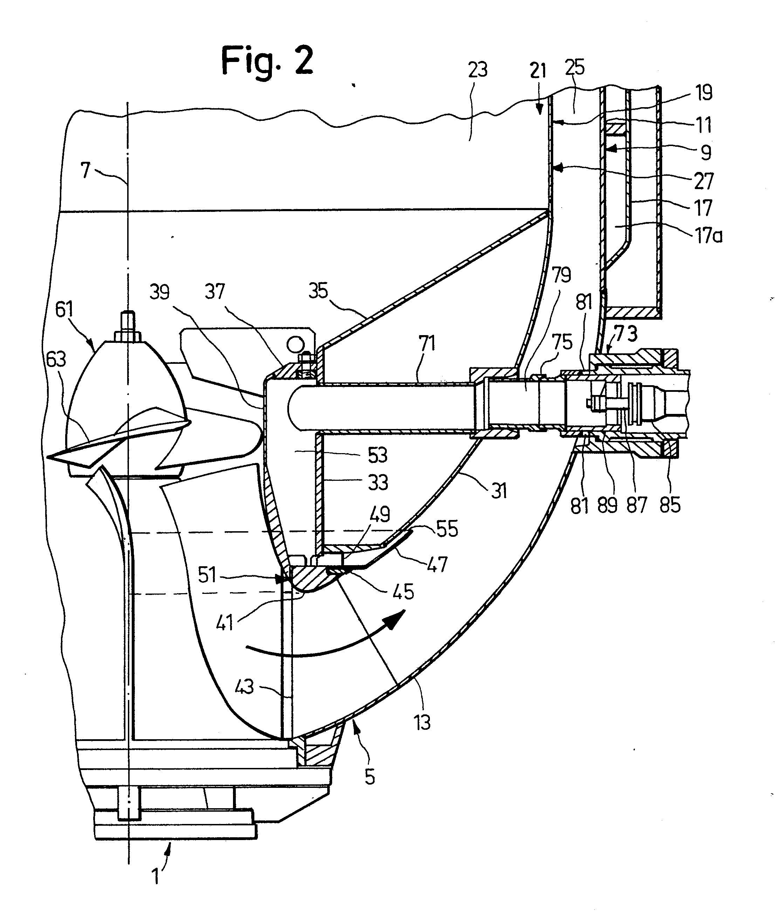

In der Kammer 5 ist eine als Ganzes mit 19 bezeichnete, mehrteilige, etwas vereinfacht dargestellte, mindestens im allgemeinen zur Kammer-Wandung 9 koaxiale Trennwand 19 angeordnet. Die Trennwand 19 unterteilt den mittleren und unteren Höhenbereich des Innenraums 21 der Kammer 5 in einem ersten, inneren, einen vollen Querschnitt aufweisenden Teilraum 23 und einen zweiten äusseren, im Querschnitt ringförmigen Teilraum 25. Die Trennwand 19 weist einen oben offenen, zylindrischen Abschnitt 27 mit nach innen ragendem Überström-Rand 29 auf. An das untere Ende des Abschnitts 27 schliesst ein sich nach unten an die Achse 7 annähernder, im Vertikalschnitt gebogener End- oder Bodenabschnitt 31 an. Dieser ist, wie man besonders deutlich aus der Figur 2 ersehen kann, bei seinem unteren, horizontalen, zur Achse 7 hin ragenden Rand dicht und starr mit einem nach oben ragenden, hohlzylindrischen Mantel 33 verbunden, dessen oberer Rand durch ein nach oben und aussen geneigtes, konisches Leitelement 35 dicht mit der Trennwand 19 verbunden ist, und zwar ungefähr bei der Verbindungsstelle von deren Abschnitten 27 und 31. An einem in der Nähe des oberen Endes des Mantels 33 nach innen vorstehenden Ansatz ist ein Flansch 37 mindestens einigermassen dicht festgeschraubt, dessen Innenrand ein nach unten ragender Hals 39 starr an befestigt ist. An das untere Ende des Halses 39 schliesst mehr oder weniger dicht ein nach aussen ragender Tragring 41 an, der mit beispielsweise vier über seinen Umfang verteilten, fussartigen Stützen 43 starr mit dem End- oder Bodenabschnitt 13 der Wandung 9 verbunden ist. Der Tragring 41 ist bei seinem Aussenrand auf seiner oberen Seite mit einer Kehle versehen, in die ein Distanzring 45 und auf diesem ein Düsenring 47 eingelegt sind. Am unteren Rand des Abschnitts 31 der Trennwand 19 sind über dessen Umfang verteilte, fussartige Klemmstücke 49 starr befestigt, die auf den Düsenring 47 drücken und diesen am Tragring 41 festklemmen. Der Mantel 33, der Flansch 37, der Hals 39 und die Ringe 41, 45, 47 bilden zusammen einen Gasverteiler 51 mit einem ringförmigen Hohlraum 53. Der Düsenring 47 hat einen nach aussen und oben geneigten, konischen Randabschnitt, der mindestens ungefähr die gleiche Neigung wie der sich über ihm befindende Teil des End- oder Bodenabschnitts 31 hat und mit diesem zusammen die ringförmige, die Achse 7 umschliessende Mündung 55 des Gasverteilers 51 bildet.

Der zylindrische Abschnitt 27 der Trennwand 19 weist einen untern Teilabschnitt, der zusammen mit dem End- oder Bodenabschnitt 31 einen feststehenden Trennwandteil bildet, und einen oberen Teilabschnitt auf. Dieser hat einen etwas grösseren Durchmesser als der untere Teilabschnitt und ist auf dessen Aussenseite mittels eines an dessen oberem Ende angeordneten, aus dem unter dem Handelsnamen Teflon erhältlichen Kunststoff bestehenden Ring vertikal verschiebbar geführt und bildet zusammen mit dem Überström-Rand 29 einen höhenverstellbaren Trennwandteil, wobei die beiden Trennwandteile durch den Kunststoff-Ring mindestens einigermassen dicht gegeneinander abgedichtet sind, sodass das beim Betrieb durch den zweiten Teilraum 25 nach oben geförderte Substrat zumindest zum grössten Teil oder eventuell vollständig bis zum Überström-Rand 29 strömt. Der feststehende Trennwandteil ist mit nicht dargestellten Schrauben derart lösbar an der Wandung 9 befestigt, dass die Trennwand bei gelösten Schrauben nach oben aus der Kammer 5 heraushebbar ist.

Eine Fördervorrichtung 61 weist ein Förderorgan 63, nämlich ein um die Achse 7 drehbar im Hals 39 angeordnetes Schaufelrad, einen zum Antreiben von diesem dienenden, unter dem End- oder Bodenabschnitt 13 angeordneten Motor 65 sowie ober- und unterhalb des Schaufelrades starr mit dem Hals 39 verbundene, schaufelartige Leitelemente auf.

Der Hohlraum 53 des Gasverteilers 51 ist oberhalb der Mündung 55 mit einer Gaszuleitung 71 verbunden, die einen horizontal und radial vom Mantel 33 weg nach aussen verlaufenden Abschnitt aufweist, der die End- oder Bodenabschnitte 31 und 13 der Trennwand 19 bzw. Wandung 9 durchdringt und im Bereich der Durchdringungstelle der Wandung 9 ein Ventil 73 aufweist. Dieses hat ein mehrteiliges, ein wenig vereinfacht gezeichnetes, zum Teil an der Wandung 9 und zum Teil an der Trennwand 19 befestigtes Gehäuse 75, das zusammen mit einem in ihm angeordneten Zapfen 85 einen den Einlass 77 des Ventils 73 mit der Mündung 55 des Gasverteilers 51 verbindenden Durchgang 79 begrenzt. Das Gehäuse 75 hat in der Nähe der Stelle, bei der es die Wandung 9 durchdringt, mindestens ein Loch, nämlich über seinen Umfang verteilte, schlitzförmige Löcher, die je eine Abzweigung 81 des Durchgangs 79 bilden und diesen mit dem zweiten Teilraum 25 verbinden. Eine vereinfacht dargestellte, am Ventilgehäuse 75 angeordnete Stellvorrichtung 83 besitzt einen Motor sowie manuell betätigbare Stellmittel und ermöglicht, über eine den Zapfen 85 durchdringende Stange 87 ein hülsenförmiges, verschiebbar im Gehäuse 75 geführtes Sperrorgan 89 in axialer Richtung zwischen zwei Stellungen hin und her zu verschieben. Wenn sich das Sperrorgan 89 in seiner in der Figur 2 gezeichneten, ersten Stellung befindet, gibt es den Durchgang 79 frei und sperrt die Abzweigung 81. Wenn das Sperrorgan 89 nach rechts in seine zweite Stellung verschoben wird, umgreift es den Zapfen 85 und sperrt dadurch den Durchgang 79, gibt dafür aber die Abzweigungen 81 frei, so dass der sich von der Mündung 55 bis zum Sperrorgan 89 erstreckende Leitungs- und Durchgangsteil durch die Abzweigungen 81 mit dem Innenraum 21 verbunden wird. Der sich ausserhalb der Kammer 5 befindende Teil des Durchgangs 79 ist auf seiner unteren Seite noch mit einem Auslass 91 verbunden, der mit einem Ventil 93 absperrbar ist.

Der Reaktor 1 weist noch einen Gasauslass 101, einen Substrataulass 103 mit einem Absperrorgan 105, einen gleichzeitig als Guckloch dienenden, mit einem Verschluss abschliessbaren Substrateinlass 107 und mindestens eine Niveau-Sonde 109 auf. Ferner können nach Stellmittel vorhanden sein, um die Höhe des oberen, den höhenverstellbaren Überström-Rand 29 aufweisenden Trennwandteils zu verstellen. Die Stellmittel können zum Beispiel mindestens ein muskelkraftfrei arbeitendes, zum Beispiel elektrisch oder hydraulisch antreibbares Sellorgan aufweisen. Ferner ist vorzugsweise eine elektrisch mit der bzw. jeder Niveau-Sonde und dem bzw. jedem Stellorgan verbundene, elektronische Steuer- und Regelvorrichtung vorhanden.

Wenn in der Kammer 5 eine mikrobiologische Kultur behandelt, insbesondere vermehrt und kultiviert werden soll, wird eine in die Kammer 5 eingebrachte Charge von flüssigem Substrat 121 mitsamt den darin vorhandenen Mikroorganismen mit der Fördervorrichtung 61 umgewälzt. Dabei pumpt das Förderorgan 63 das Substrat 121 aus dem ersten Teilraum 23 durch den Hals 39 hindurch nach unten. Das Substrat gelangt dann nach einer Umleitung beim unteren Halsende in den zweiten Teilraum 25 und strömt durch diesen hindurch nach oben, bis es über den Überström-Rand 29 wieder in den ersten Teilraum 23 zurückfällt. Wenn während dieser Umwälzung des Substrats 121 ein aerober, mikrobiologischer Prozess in diesem stattfinden soll, wird durch die Gaszuleitung 71 Luft oder eventuell Sauerstoff zugeführt, wobei sich das Sperrorgan 89 in seiner ersten, in der Figur 2 gezeichneten Stellung befindet. Die Luft oder der Sauerstoff gelangt dabei zum Gasverteiler 51, strömt durch dessen ringspaltförmige Mündung 55 geneigt nach oben in den zweiten Teilraum 25 und vermischt sich dort mit dem umgewälzten Substrat. Dieses bildet nach der Umlenkung beim unteren Ende des Halses 39 normalerweise eine stark turbulente Strömung, was eine gute und gleichmässige Verteilung des in das Substrat eingeleiteten Gases fördert. Da das Gas erst in das Substrat 121 eingeleitet wird, wenn dieses das Förderorgan 63 passiert hat und zum Überström-Rand 29 strömt, kann man ohne weiteres verhältnismässig grosse Gasmengen pro Zeiteinheit in das Substrat einleiten, ohne dass dadurch die Förderwirkung des Förderorgans 63 gestört wird. Dadurch wird auch bei grosser Gaszufuhr eine kontinuierliche und gleichmässige Umwälzung des Substrats 121 sowie eine gleichmässige Gaseinleitung in dieses ermöglicht. Im übrigen kann die Kammer 5 während der Durchführung des mikrobiologischen Prozesses durch Hindurchleiten einer Kühlflüssigkeit durch den Durchgang 17a der Vorrichtung 17 gekühlt werden.

Wenn der Innenraum 21 der Kammer 5 und die Gaszuleitung 71 nach der Durchführung eines Prozesses, beispielsweise zum Wechseln der Mikroorganismen-Kultur, steril gemacht werden soll, bringt man das Sperrorgan 89 des Ventils 73 in seine zweite Stellung, in der es den Durchgang 79 auf der Aussenseite der Kammer 5 sperrt und den sich im Kammer-Innenraum 21 befindenden Durchgangsteil durch die Abzweigungen 81 mit dem Kammer-Innenraum 21 verbindet. Das für den Sterilisationsvorgang im letzteren belassene, flüssige Substrat kann nun in den Hohlraum 53 des Gasverteilers und den Durchgang der Gaszuleitung 71 und des Ventils 73 strömen und die Gaszuleitung 71 von der Mündung 55 bis zum Sperrorgan 89 füllen. Die Kammer 5 kann durch Hindurchleiten eines Heizfluids durch den Durchgang 17a der Vorrichtung auf ungefähr 120°C erhitzt werden und dadurch mitsamt dem Gasverteiler 51 sowie dem im sich im Innenraum 21 befindenden Teil der Gaszuleitung 71 steril gemacht werden. Damit auch der äussere Teil der Gaszuleitung 71 steril wird, leitet man Wasserdampf in diesen ein. Das nach dessen Abkühlung aus diesem entstehende Kondensat kann durch vorübergehendes Öffnen des Ventils 93 aus dem Durchgang 79 abgeleitet werden.

Die Einrichtung mit dem Reaktor 1 kann für einen grosse Anzahl verschiedener, aerober, mikrobiologischer Prozesse verwendet werden. Im Reaktor kann beispielsweise aus Kohlenwasserstoff mittels Mikroorganismen Protein hergestellt werden, wobei der Kohlenwasserstoff dem Substrat in Form von Methanol zugegeben werden kann, das als Nährstoff für die Mikroorganismen dient. Des weiteren können zum Beispiel auch Hefe, Vitamin C oder Enzyme durch mikrobiologische Umsetzungsprozesse hergestellt werden. Dabei können je nach der Art des Prozesses zusätzlich zur Kultivierung von Mikroorganismen auch noch andere Prozesse, etwa chemische Reaktionen, an denen ein von den Mikroorganismen erzeugtes Produkt beteiligt ist, im Reaktor stattfinden.

Falls mit dem Reaktor 1 Prozesse durchgeführt werden sollen, für die stark voneinander abweichende Luftzufuhrmengen erforderlich sind, kann man nötigenfalls zur Anpassung die Breite des die Gasverteiler-Mündung 55 bildenden Ringspalts verändern, indem man den Distanzring 45 durch einen dickeren oder dünneren Distanzring ersetzt und eventuell auch den Düsenring 47 auswechselt. Zum Auswechseln des Distanzrings 45 und eventuell des Düsenrings 47 können der Deckel 15 entfernt sowie die nicht dargestellten Schrauben, mit denen die Trennwand 19 mit der Kammer-Wandung 9 verschraubt ist, gelöst werden und die Trennwand 19 vorübergehend aus der Kammer 5 herausgehoben und dadurch vom Düsenring 47 abgehoben werden.

Der Düsenring 47 ist beispielsweise ausreichend formfest, dass der die Mündung 55 bildende Ringspalt unabhängig von den momentanen Strömungs- und Druckwerten immer die gleiche Breite hat. Man könnte jedoch eventuell auch vorsehen, den Düsenring derart federnd auszubilden, dass er beim Hindurchleiten von Luft oder Sauerstoff durch den Gasverteiler flattert, so dass die Mündung 55 abwechselnd enger und weiter wird.

Beim Betrieb des Reaktors bildet sich auf dem im innern, ersten Teilraum 23 vorhandenen Substrat normalerweise eine Schaumschicht. Die Niveau-Sonde 109 ermittelt laufend bei der Kammer-Mittelachse das Niveau des Schaums, d.h. die Höhe der Grenzfläche, die die Schicht des aus Substrat und Gasblasen bestehenden Schaums oben begrenzt. Falls kein Schaum vorhanden sein sollte, ermittelt die Sonde 109 das Niveau des flüssigen Substrats. Die Sonde 109 kann das erfasst Niveau in Form elektrischer, analoger oder digitaler Signale an die Steuer- und/oder Regelvorrichtung übermitteln. Wenn die Mikroorganismen sich im Verlauf ihrer Kultivierung vermehren und eventuell nebst Gasen auch flüssige oder feste Stoffe an das Substrat abgeben, vergrössert sich im allgemeinen das von diesem eingenommene Volumen. Die Steuer- und/ oder Regelvorrichtung steuert und/oder regelt das bwz. jedes Stellorgan der Stellmittel in Abhängigkeit des mit der Niveau-Sonde 109 ermittelten Niveaus derart, dass der Überström-Rand 29 immer um eine Höhendifferenz über dem Niveau des Schaum bzw. beim allfälligen Fehlen von Schaum über dem Niveau des Substrates liegt und dass diese Höhendifferenz, mindestens gleich einem zur Schaumzerstörung notwendigen Mindestwert ist, sodass der im ersten, inneren Teilraum 23 vorhandene Schaum und natürlich auch das im ersten Teilraum 23 vorhandene Substrat während der ganzen Zeitdauer, in der das Substrat in der Kammer 5 umgewälzt und/oder Gas in dieses eingeleitet wird, nie über den ersten Teilraum hinaussteigen. Wenn also das Volumen des suspendierte Mikroorganismen und eventuell noch von diesen produzierte Stoffe enthaltenden Substrats zunimmt, heben die Stellmittel den höhenverstellbaren, den Überström-Rand 29 aufweisenden Trennwandteil nach oben. Für weitere Einzelheiten der Ausbil dung und Arbeitsweise der zur Verstellung des höhenverstellbaren Trennwandteils dienenden Mittel sei auf die gleichzeitig der Schweizerpatentanmeldung 183/86 vom 17. Januar 1986 eingereichte, europäische Patentanmeldung (Veröffentlichung EP-A-     ) verwiesen.

Die Einrichtung könnte noch in anderer Hinsicht geändert werden.

Beispielsweise könnte man den Gasverteiler statt mit nur einer einzigen, ringspaltförmigen Mündung mit mehreren Mündungen versehen. Diese könnten zum Beispiel durch zwei oder mehr die Achse 7 umschliessende Ringspalte oder durch um die Achse 7 herum verteilte Löcher gebildet sein.

Des weitern könnte man den Hals der Trennwand nach unten bis zum Boden der Kammer-Wandung verlängern und ihn dafür mit über seinen Umfang verteilten, zur Achse 7 radialen Durchgängen versehen. Anstelle des Förderorgans 63 könnte dann in jedem dieser radialen Durchgänge ein durch ein Schaufelrad gebildestes Förderorgan angeordnet sein. Das sauerstoffhaltige Gas, d.h. die Luft oder der Sauerstoff, würde dann mit dem Gasverteiler zwischen den einander strömungsmässig parallel geschalteten Förderorganen und dem Überström-Rand in das umgewälzte Substrat eingeleitet.

Insbesondere bei verhältnismässig kleinen Reaktoren könnte der beim in der Figur 1 dargestellte Reaktor am oberen Ende des höhenverstellbaren Trennwandteils vorhandene, radial nach innen ragende Kragen wegfallen, sodass der Überström-Rand dann durch den oberen Rand des zylindrischen Mantels des höhenverstellbaren Trennwandteils gebildet würde.

**Patentansprüche**

1. Einrichtung zum Behandeln, insbesondere Kultivieren, von Mikroorganismen in einem flüssigen Substrat, mit einer Kammer (5), einer in dieser angeordneten, deren Innenraum (21) in zwei Teilräume (23, 25) unterteilenden, am oberen Ende einen Überström-Rand (29) bildenden Trennwand (19), einem Förderorgan (63), um das Substrat aus dem untern Ende des einen, ersten Teilraums (23) durch den anderen, zweiten Teilraum (25) hindurch nach oben zu fördern, so dass es über den Überström-Rand (29) in den ersten Teilraum (23) zurückströmt, einer Gaszuleitung (71) und einem mit dieser verbundenen, mit mindestens einer Mündung (55) in den Kammer-Innenraum (21) mündenden Gasverteiler (51), dadurch gekennzeichnet, dass sich die mindestens eine Mündung (55), bezogen auf die Strömungsrichtung des Substrates, zwischen dem Förderorgan (63) und dem Überström-Rand (29) befindet.

2. Einrichtung nach Anspruch 1, dadurch gekennzeichnet, dass als Mündung (55) ein die Achse (7) der Kammer (5) umschliessender Ringspalt vorhanden ist.

3. Einrichtung nach Anspruch 1 oder 2, wobei der zweite Teilraum (25) den ersten Teilraum (23) im Querschnitt ringförmig umschliesst, dadurch gekennzeichnet, dass sich die mindestens eine Mündung (55) im zweiten Teilraum befindet.

4. Einrichtung nach Anspruch 3, wobei die Wandung (9) der Kammer (5) sowie die Trennwand (19) sich zu ihren unteren Enden hin an die Achse (7) der Kammer (5) annähernde, vorzugsweise im Vertikalschnitt zumindest stellenweise gebogene Endabschnitte (13, 31) aufweisen, dadurch gekennzeichnet, dass die mindestens eine Mündung (55) sich zwischen den beiden genannten Endabschnitten der Kammer-Wandung (9) und der Trennwand (19) befindet.

5. Einrichtung nach Anspruch 4, dadurch gekennzeichnet, dass die Mündung (55) von der Achse (7) der Kammer (5) weg nach oben geneigt ist.

6. Einrichtung nach einem der Ansprüche 1 bis 5, dadurch gekennzeichnet, dass die mindestens eine Mündung (55) auf einer Seite durch die Trennwand (19) und/oder ein fluiddicht mit dieser verbundenes Element begrenzt ist.

7. Einrichtung nach einem der Ansprüche 1 bis 6, dadurch gekennzeichnet, dass die Mündung (55) durch lösbar miteinander verbundene Teile (31, 47) begrenzt ist.

8. Einrichtung nach einem der Ansprüche 1 bis 7, wobei der erste Teilraum (23) an seinem unteren Ende mit dem zweiten Teilraum (25) durch einen zur Achse (7) der Kammer (5) koaxialen Hals (39) verbunden ist, in dem das beispielsweise als Schaufelrad ausgebildete Förderorgan (63) angeordnet ist, dadurch gekennzeichnet, dass der Hals (39) zusammen mit einem ihn umschliessenden Mantel (33) einen ringförmigen Hohlraum (53) begrenzt, in den die Gaszuleitung (71) mündet und der mit der Mündung (55) des Gasverteilers (51) verbunden ist.

9. Einrichtung nach einem der Ansprüche 1 bis 8, dadurch gekennzeichnet, dass die Gaszuleitung (71) oberhalb der mindestens einen Mündung (55) in den Kammer-Innenraum (21) eindringt und im Bereich der Durchdringungstelle ein Ventil (73) mit einem Einlass (77), einem diesen mit der mindestens einen Mündung (55) verbindenden Durchgang (79) und mindestens einer von diesem in den Kammer-Innenraum (21) mündende Abzweigung (81) aufweist und dass das Ventil (73) ausgebildet ist, um die mindestens eine Mündung (55) in einer Stellung mit dem Einlass (77) und in einer anderen Stellung mit der Abzweigung (81) zu verbinden.

10. Einrichtung nach einem der Ansprüche 1 bis 9, dadurch gekennzeichnet, dass der Überström-Rand (29) höhenverstellbar ist, wobei die Trennwand (19) zum Beispiel einen unverschiebbar befestigten, zylindrischen Abschnitt und einen auf dessen Aussenseite vertikal verschiebbar geführten, zylindrischen Abschnitt aufweist, der an seinem oberen Ende mit dem Überström-Rand (29) versehen ist, und wobei zum Beispiel mindestens eine Niveau-Sonde (109), um das Niveau des im ersten Teilraum (23) oben auf dem in diesem enthaltenen Substrat vorhandenen Schaums oder, im Fall der Abwesenheit von Schaum, das Niveau des Substrats zu ermitteln, eine Stellvorrichtung zum vertikalen Verstellen des Überström-Randes (29) und eine elektrisch mit der Niveau-Sonde (109) sowie der Stellvorrichtung verbundene Steuer- und Regelvorrichtung vorhanden sind, um die Höhe des Überström-Randes (29) in Abhängigkeit vom mit der Niveau-Sonde (109) ermittelten Schaum- bzw. Substrat-Niveau einzustellen.

Fig.1

0236259

Fig. 2

0236259